# EUROPÄISCHE PATENTANMELDUNG

(11) **EP 2 862 594 A1**
(43) Veröffentlichungstag der Anmeldung: **22.04.2015**
(21) Anmeldenummer: 14186735.8
(22) Anmeldetag: 29.09.2014
(51) Int. Cl.: A61N 1/05

(54) **Aktive, reversible Fixierung von CRT Elektroden**

(30) Priorität: 17.10.2013 US 201361891921 P
(71) Anmelder: BIOTRONIK SE & Co. KG, 12359 Berlin (DE)
(72) Erfinder: Weitzig, Pierre, 54666 Irrel (DE); Jadwizak, Detmar, 15537 Erkner (DE); Hillebrand, Gordon, 12157 Berlin (DE); Fründt, Carsten, 13057 Berlin (DE)
(74) Vertreter: Lindner-Vogt, Karin L.

(57) **Zusammenfassung**

Implantierbare medizinische Elektrodenvorrichtung, insbesondere kardiovaskuläre Herzschrittmacher-Elektrodenvorrichtung, angepasst zum Einsetzen in ein Blutgefäß, umfassend eine langgestreckte Elektrodenleitung mit einem schlauchartigen Elektrodenkörper, der einen vom proximalen Ende zum distalen Ende durchgehenden Hohlraum mit einem Innendurchmesser aufweist und an dessen distalem Ende eine Elektrode und an dessen proximalem Ende ein Elektrodenanschluss vorgesehen ist, und einer Fixierungsvorrichtung mit einer Ballonkathetereinrichtung, die einen langgestreckten schlauchartigen, hohlen Katheterkörper mit einem Außendurchmesser, der kleiner als der Innendurchmesser des Hohlraums des Elektrodenkörpers ist, und einem inflatierbaren Ballon an oder nahe dem distalen Ende, mit einem im deflatierten Zustand gegenüber dem Innendurchmesser des Hohlraums des Elektrodenkörpers kleineren Außendurchmesser, und einem Fluidanschluss am proximalen Ende, der an den Elektrodenanschluss des Elektrodenkörpers angepasst ist, Verbindungsmitteln zur lösbaren Verbindung der Elektrodenleitung mit der Fixierungsvorrichtung und Dichtungsmitteln zum fluiddichten Verschluss des Katheterkörpers der Fixierungsvorrichtung in ihrem Gebrauchszustand mit inflatiertem Ballon.

## Beschreibung

Die Erfindung betrifft eine implantierbare medizinische Elektrodenvorrichtung, insbesondere kardiovaskuläre Herzschrittmacher-Elektrodenvorrichtung, angepasst zum Einsetzen in ein Blutgefäß, umfassend eine langgestreckte Elektrodenleitung mit einem schlauchartigen Elektrodenkörper, der einen vom proximalen Ende zum distalen Ende durchgehenden Hohlraum mit einem Innendurchmesser aufweist und an dessen distalem Ende eine Elektrode und an dessen proximalem Ende ein Elektrodenanschluss vorgesehen ist, und eine Fixierungsvorrichtung zur Festlegung der Position im Blutgefäß. Sie betrifft des Weiteren eine Fixierungsvorrichtung für eine solche Elektrodenvorrichtung sowie ein entsprechendes medizinisches Elektrodenimplantationssystem.

Ein grundsätzliches Erfordernis beim Einsatz und somit bei der Konstruktion implantierbarer Elektrodenleitungen ist die Gewährleistung ihrer korrekten Position in einem Hohlorgan oder Gefäß des Körpers, in dem sie ihre Wirkung entfalten sollen. Daher ist die Entwicklung und ständige Verbesserung geeigneter Fixierungsvorrichtungen seit vielen Jahren untrennbarer Bestandteil der technischen Entwicklung auf diesem Gebiet und es ist eine Vielzahl unterschiedlicher Fixierungsmechanismen bekannt.

Speziell bei sogenannten CRT-Elektroden (Elektrodenleitungen für biventrikuläre oder 3-Kammer-Schrittmacher) werden unter Anderem Noppen oder Finnen (tines) am Umfang des distalen Elektrodenendes, aus dem Elektrodenende herausstehende Silikonschrauben oder Helices oder auch vorgeprägte J- oder S-förmige Krümmungen des distalen Endabschnitts der Elektrodenleitung als passive Fixierungsmittel oder beispielsweise ein geschlitzter und am Einsatzort manuell gestauchter Schlauch als aktives Fixierungsmittel verwendet.

In der WO 94/07564 A1 wird eine Lösung für das Problem der Umplatzierbarkeit offenbart, bei der die Fixierung durch einen expandierbaren oder selbst expandierenden Drahtkorb erfolgt. Nachteil dieser Konstruktion ist, dass die Drähte in die Gefäßwand einschneiden und somit sehr traumatisch sein können, und dass sie nicht reversibel explantierbar ist.

Weiterhin wird auf die US 2006/0036307 A1 hingewiesen. Diese offenbart eine implantierbare kardiovaskuläre Herzschrittmacher- oder Defibrillator-Elektrodenvorrichtung, die einen langgestreckten, schlauchartigen Elektrodenkörper und eine Fixierzone im distalen Endbereich des Elektrodenkörpers umfassen. Die Fixierzone hat eine nach außen geschlossene Umfangshülle und dient der lösbaren Fixierung der Elektrodenvorrichtung in einem Körper-Lumen, indem sie reversibel expandierbar ist. Die Steuerung der Expansion oder Kontraktion wird durch ein in der Fixierzone angeordnetes Expansionsmittel gewährleistet.

Zusätzlich zu diesen Merkmalen zeigt die EP 0 546 414 A1 eine Elektrodenvorrichtung, bei der das Expansionsmittel durch pneumatische oder hydraulische Druckbeaufschlagung gebildet ist, mit deren Hilfe die in der Fixierzone flexible Wandung des Elektrodenkörpers reversibel expandierbar ist.

Die EP 1 832 309 B1 der Anmelderin zeigt eine weitere implantierbare medizinische Elektrodenvorrichtung, speziell Herzschrittmacher- oder Defibrillator-Elektrodenvorrichtung, bei der das Fixierungsproblem bis zu einem gewissen Grade durch das Vorsehen einer stentartigen, plastisch verformbaren Stützstruktur in der Wandung des Elektrodenkörpers gelöst wird. Diese Stützstruktur ist durch Beaufschlagung mit Überdruck in einen stationär expandierten Fixierzustand und durch Beaufschlagung mit Unterdruck in einen kontrahierten Ausgangszustand überführbar. Hierbei handelt es sich um eine spezielle, integrierte und konstruktiv recht aufwändige Elektrodenleitungs-Konstruktion, die nicht reversibel explantierbar ist.

Aus der WO 2010/138648 A1 ist es bekannt, einen Führungsdraht mit Hilfe eines in diesen integrierten expandierbaren Ballons in einem Gefäß zu fixieren und anschließend, in an sich bekannter Weise, über den Führungsdraht eine Elektrodenleitung zum gewünschten Punkt des Gefäßes, an dem der Führungsdraht mittels des Ballons fixiert ist, vorzuschieben.

Bei den meisten gängigen passiven Fixierungen ist die Anpresskraft an die Gefäßwand und somit die Fixierung in der gewünschten Position von der vom Elektrodenhersteller entwickelten Vorformung der Elektrode im distalen Bereich abhängig.

Vorformungen durch Wärme oder durch mechanische Belastung können die Wendeleigenschaften bzgl. der Langzeitstabilität negativ beeinflussen. Die Qualität (bzw. die Anpresskräfte) der Vorformungen durch Wärme oder mechanische Belastungen schwanken sehr stark aufgrund von z.B. unterschiedlichen Drahtchargen. Passive Fixierungen sind oft nicht für die große Varianz an Gefäßdurchmessern geeignet. Fixierungen wie z.B. die Silikonschraube und Noppen sind für kleine Gefäße (Zielvenen), J-Bogen, Helix oder S-Kurve sind für größere Gefäße geeignet (Koronarsinus oder Abgänge des Koronarsinus). Dies hat zur Folge, dass die Elektrode nicht immer an ihrer optimalen Position mit einer guten Reizschwelle platziert werden kann. Die Dislokationsrate einer CRT Elektrode ist außerdem wesentlich höher als bei Rechtsherz-Elektroden, die im Atrium oder im Ventrikel implantiert werden. Zusätzlich besteht die Gefahr, dass beim Entfernen (Slitten) des Führungskatheters die gerade gelegte Elektrode disloziert.

In einigen Fällen wird eine ungünstige Position mit einer schlechten Reizschwelle in Kauf genommen, weil eine Linksherz-Elektrode nicht sicher in ihrer optimalen Zielvene fixiert werden kann.

Die Funktion einer Fixierung, eine gewisse Steifigkeit zu besitzen um sich gut an die Gefäßwand anzudrücken, steht im Widerspruch zur Flexibilität, Weichheit und Manövrierbarkeit während der Implantation.

Aktive Fixierungen haben den großen Nachteil, nicht explantierbar zu sein. Das Prinzip besteht darin, unregelmäßige Verdickungen, Verankerungen oder Materialanhäufungen während der Implantation zu erzeugen. Die Elektrode ist dadurch nicht mehr isodiametrisch, die Fixierelemente wachsen fest ein, was eine Explantation unmöglich macht. Z.B. bei Infektionen ist dies aber Lebensnotwendig.

Der Erfindung liegt mithin die Aufgabe zu Grunde, eine in ihren Gebrauchseigenschaften unter verschiedenen Bedingungen verbesserte und erforderlichenfalls unproblematisch explantierbare Elektrodenvorrichtung bereitzustellen.

Diese und weitere Aufgaben werden durch eine Elektrodenvorrichtung mit den Merkmalen des Anspruchs 1 gelöst. Des Weiteren wird ein medizinisches Elektrodenimplantationssystem mit den Merkmalen des Anspruchs 9 bereitgestellt.

Zur Erfindung gehören die folgenden Überlegungen der Erfinder:

Diese Fixierung soll Dislokationen einer platzierten Elektrode drastisch minimieren. Sie darf sich erst nach der Implantation entfalten und soll größer als der Elektrodendurchmesser sein. Sie muss sich sicher in Koronarvenen mit verschiedenen Durchmessern verankern.

Diese Fixierung ist optional zu verwenden und kann zusätzlich mit jeder auf dem Markt gängigen CRT-Elektrode kombiniert/ergänzt werden. Zudem soll die Fixierung eine eventuelle Repositionierung oder Explantierung der Elektrode nicht erschweren. Das Fixierungselement soll im "wirklosen Zustand" geringeren Durchmesser als der Elektrodenleitungskörper haben. Das Fixierungselement soll unabhängig vom Elektrodendesign und von den verwendeten Materialien sein.

Diese Überlegungen führen zum Vorsehen eine Fixierungsvorrichtung mit einer Ballonkathetereinrichtung, die einen langgestreckten schlauchartigen, hohlen Katheterkörper mit einem Außendurchmesser, der kleiner als der Innendurchmesser des Hohlraums des Elektrodenkörpers ist, einen inflatierbaren Ballon an oder nahe dem distalen Ende, mit einem im deflatierten Zustand gegenüber dem Innendurchmesser des Hohlraums des Elektrodenkörpers kleineren Außendurchmesser, und einen Fluidanschluss am proximalen Ende hat, der an den Elektrodenanschluss des Elektrodenkörpers angepasst ist. Des Weiteren gehört zur Erfindung das Vorsehen von Verbindungsmitteln zur lösbaren Verbindung der Elektrodenleitung mit der Fixierungsvorrichtung und Dichtungsmitteln zum fluiddichten Verschluss des Katheterkörpers der Fixierungsvorrichtung in ihrem Gebrauchszustand mit inflatiertem Ballon.

In ihrer Handhabung stellt sich die erfindungsgemäße Elektrodenvorrichtung wie folgt dar:

In einer Ausführung der Erfindung ist am proximalen Ende der Fixierungsvorrichtung ein Steckeradapter angebracht, der innenseitig zur Aufnahme des Elektrodenanschlusses der Elektrodenleitung und außenseitig zum Einführen in eine Elektrodenbuchse eines elektromedizinischen Gerätes, insbesondere Herzschrittmachers, sowie zum Einführen einer Injektionskanüle in den Fluidanschluss des Katheterkörpers angepasst ist. In Ausgestaltungen der Erfindung, die speziell auf CRT-Elektroden zugeschnitten sind, ist der äußere Elektrodenanschluss des Steckeradapters als IS-1 oder IS-4 Elektrodenanschluss ausgebildet.

Die Elektrodenleitung (im Prinzip eine beliebige CRT-Elektrode) wird nach herkömmlicher Methode implantiert. Nach dem Entfernen des Guidewires oder des Stylets kann die Ballonkathetereinrichtung ("Ballonfixierung") eingeführt werden. Der Elektrodenstecker wird dabei in den Steckeradapter an dem Schlauch des Fixierungsballons eingeschoben. Mit Hilfe einer passenden Kanüle und Handspritze kann der Ballon mit einem flüssigen Medium inflatiert werden, um die Elektrode zu fixieren. Durch die Dichtungsmittel im Steckeradapter wird ein selbstständiges Deflatieren des Fixierungsballons verhindert. Abschließend wird der Adapter zusammen mit der Elektrode in die Kavität des Schrittmacher-oder Defibrillatorgehäuses geschoben und verankert.

Bei einer möglichen Umpositionierung oder Explantation kann der Adapter mit Elektrode aus dem Gehäuse des Gerätes herausgezogen werden, und die Kanüle mit der Handspritze eingeschoben werden, um die Flüssigkeit aus dem Ballon zu entfernen bzw. den Ballon zu deflatieren.

Der Steckeradapter ist passend für entweder einen IS-1 oder für einen IS4 Stecker.

In einer weiteren Ausführung der Erfindung umfassen die Dichtungsmittel ein formelastisches zylindrisches Dichtelement, welches konzentrisch zum Katheterkörper der Ballonkathetereinrichtung angeordnet und von deren proximalem Ende her mit einer Injektionskanüle derart durchstoßbar ist, dass es sich nach dem Entfernen der Injektionskanüle wieder selbsttätig fluiddicht verschließt.

In einer weiteren Ausführung der Erfindung umfassen die Verbindungsmittel zur lösbaren Verbindung Verstellmittel, insbesondere eine in den Elektrodenkörper eingebettete und von dessen Außenumfang zugängliche, radial zum Eindringen in den Hohlraum der Elektrodenleitung und zum Eingriff mit dem schlauchartigen Katheterkörper der hierin eingesetzten Fixierungsvorrichtung ausgerichtete Fixierungsschraube zur Einstellung der longitudinalen Position der Fixierungsvorrichtung bezüglich der Elektrodenleitung.

In materialseitigen Ausführungen der Erfindung ist der Ballon der Ballonkathetereinrichtung aus einem Material aus der Silikon, PUR, ETFG, PTFE und PA umfassenden Gruppe gebildet. Im Übrigen sind für die Bestandteile der vorgeschlagenen Elektrodenvorrichtung, wie etwa die zugehörigen Schläuche, Elektroden, Zuleitungen etc., bekannte und bewährte Materialien einsetzbar. Der Fixierungsballon kann beispielsweise mit NaCl, Silikonöl oder auch mit Röntgenkontrastmittel zur zusätzlichen Erzielung einer Darstellbarkeit im Röntgenbild befüllt werden; diese Auswahl soll aber ausdrücklich nicht als beschränkend zu verstehen sein.

Die vorgeschlagene Lösung sieht in einer Ausgestaltung als modulares Elektrodenimplantationssystem insbesondere Fixierungstools mit unterschiedlicher Länge vor, die eine optimale Anpassung auf verschiedene Gefäßgrößen und gewünschte Elektrodenpositionen innerhalb eines Gefäßes erlauben, und zwar über die Einstellmöglichkeiten hinaus, die ein einzelnes Fixierungstool aufgrund inhärenter Verstellmöglichkeiten (siehe weiter unten) bietet. In diesem wichtigen Aspekt ist die modulare Lösung gemäß der vorliegenden Erfindung sämtlichen in eine Elektrodenleitung fest integrierten Fixierungsvorrichtungen grundsätzlich überlegen.

Mit der Erfindung werden, jedenfalls in einigen der oben erwähnten Ausgestaltungen, wesentliche Vorteile gegenüber bekannten Lösungen erzielt:
- Sie ist optional für jede auf dem Markt erhältliche CRT Elektrode verwendbar, (d. h. der Arzt kann während der Implantation entscheiden, ob er dieses Hilfstool verwenden möchte, z.B. bei ungenügender Fixierung der Elektrode oder ungünstigen anatomischen Bedingungen).
- Die Fixierung ist unabhängig von der Gefäßgröße.
- Die Fixierung ist unabhängig von der Anzahl der Pole einer Elektrode (uni- bi- und quattripolar).
- Die Fixierung beeinträchtigt nicht das isodiametrische Design der Elektrode.
- Die Fixierung ist unabhängig von den Materialien und dem Design der CRT-Elektrode.
- Die Dislokationsrate würde sich stark verringern.
- Die optimale Position und damit optimale Reizschwelle (Therapie) der Elektrode kann unproblematisch eingestellt werden.
- CRT-Anwendungen müssen nicht abgebrochen werden, weil keine Fixierung der Elektrode möglich ist.
- Die vorgeschlagene Elektrodenvorrichtung ist grundsätzlich explantierbar.

Vorteile und Zweckmäßigkeiten der Erfindung ergeben sich im Übrigen aus der Beschreibung eines Ausführungsbeispiels anhand der Figuren. Von diesen zeigen:
- Fig. 1: eine perspektivische Ansicht eines Ausführungsbeispiels der erfindungsgemäßen implantierbaren Elektrodenvorrichtung in einem Ausgangszustand,
- Fig. 2: eine perspektivische Ansicht eines Ausführungsbeispiels der erfindungsgemäßen implantierbaren Elektrodenvorrichtung im implantierten Zustand (Fixierungszustand),
- Fig. 3: eine perspektivische Ausschnittsdarstellung der in Fig. 1 und 2 gezeigten Elektrodenvorrichtung,
- Fig. 4: eine perspektivische Ansicht des in Fig. 3 gezeigten Vorrichtungsteils mit angesetzter Injektionskanüle und
- Fig. 5: eine Detailansicht (Längsschnittdarstellung) zum in Fig. 3 und 4 gezeigten Vorrichtungsteil, zusammen mit dem Ende der Injektionskanüle.

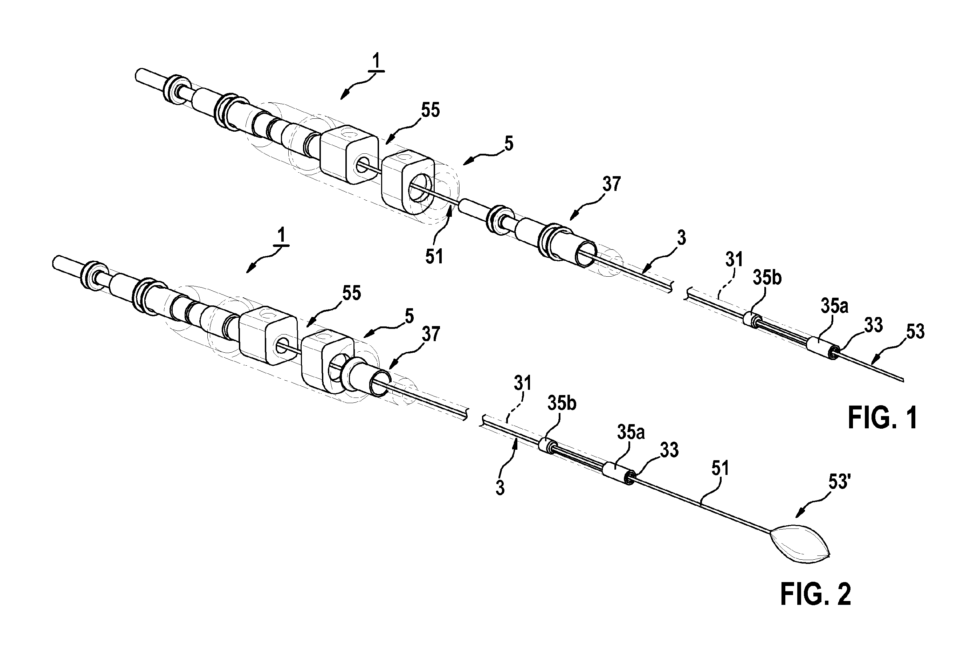

Fig. 1 und 2 zeigen jeweils eine implantierbare medizinische Elektrodenvorrichtung 1, die eine als sog. CRT-Elektrode ausgebildete Elektrodenleitung 3 und eine als Ballonkathetereinrichtung ausgebildete, separat gefertigte, im Gebrauch aber mit der Elektrodenvorrichtung verbundene Fixierungsvorrichtung 5 umfasst. Da der Aufbau der Elektrodenleitung 3 bekannt ist und Einzelheiten für die Erläuterung der Erfindung nicht von Bedeutung sind, sind die einzelnen Teile/Abschnitte in den Figuren nicht näher bezeichnet und werden nachfolgend nicht im Einzelnen beschrieben. Es wird lediglich angemerkt, dass die Elektrodenleitung 3 einen langgestreckten schlauchartigen Elektrodenkörper 31 mit einem vom proximalen Ende zum distalen Ende durchgehenden Hohlraum 33 hat und am bzw. nahe dem distalen Ende des Elektrodenkörpers zwei Elektroden 35a, 35b und am proximalen Ende ein IS-1- oder IS4 Normstecker 37 vorgesehen ist. (Alternativ kann die Elektrodenleitung auch nur eine der bis zu vier Elektroden haben.)

Die Fixierungsvorrichtung 5 umfasst einen langgestreckten schlauchartigen Katheterkörper 51 mit einem inflatierbaren Ballonabschnitt 53 (Fig. 1) bzw. inflatierten Ballon 53' am distalen Ende und einem Steckeradapter 55, dessen Innenaufbau nachfolgend genauer beschrieben wird, am proximalen Ende. In Fig. 1 ist die Fixierungsvorrichtung 5 gegenüber der Elektrodenleitung 3 etwas nach distal herausgezogen, während in Fig. 2 beide Komponenten der Elektrodenvorrichtung 1 im für den Gebrauch miteinander verbundenen Zustand und mit bereits inflatiertem Ballon 53' gezeigt sind.

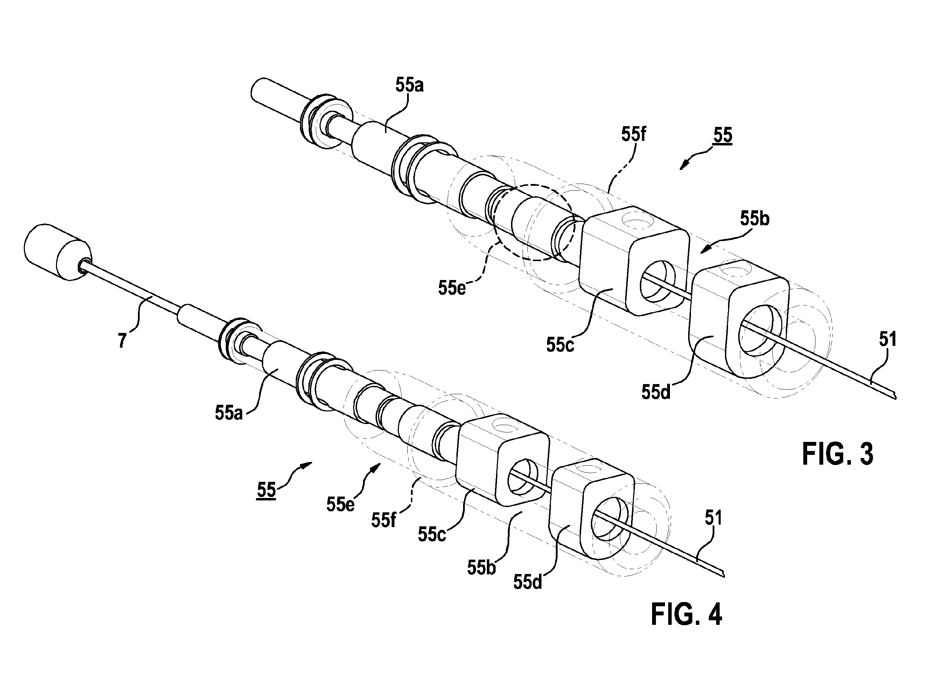

Fig. 3 und 4 zeigen, in zwei Ausschnittdarstellungen der Elektrodenvorrichtung 1, den Steckeradapter 55 der Fixierungsvorrichtung 5 genauer, und zwar in Fig. 4 mit proximal angesetzter Injektionskanüle 7 zum Befüllen des (hier nicht dargestellten) Ballons der Fixierungsvorrichtung. Der Steckeradapter 55 hat an seinem proximalen Ende einen zum Anschlussstecker 37 der Elektrodenleitung 3 baugleichen Anschlussstecker 55a, im vorliegenden Falle also vom IS-1-Typ. An seinem distalen Ende ist eine entsprechende Normbuchse (IS-1-Buchse) 55b vorgesehen. Dieser Buchsenabschnitt umfasst zwei Kontaktblöcke 55c, 55d mit jeweils einer (nicht gesondert bezeichneten) Madenschraube zum Kontaktieren und Fixieren des Steckerabschnitts der eingesetzten Elektrodenleitung (hier nicht gezeigt). Zwischen dem Steckerabschnitt 55a und dem Buchsenabschnitt 55b ist eine Dichtungseinheit 55e angeordnet, und alle genannten Teile sind in einem integralen Steckeradaptergehäuse 55f zusammengefasst.

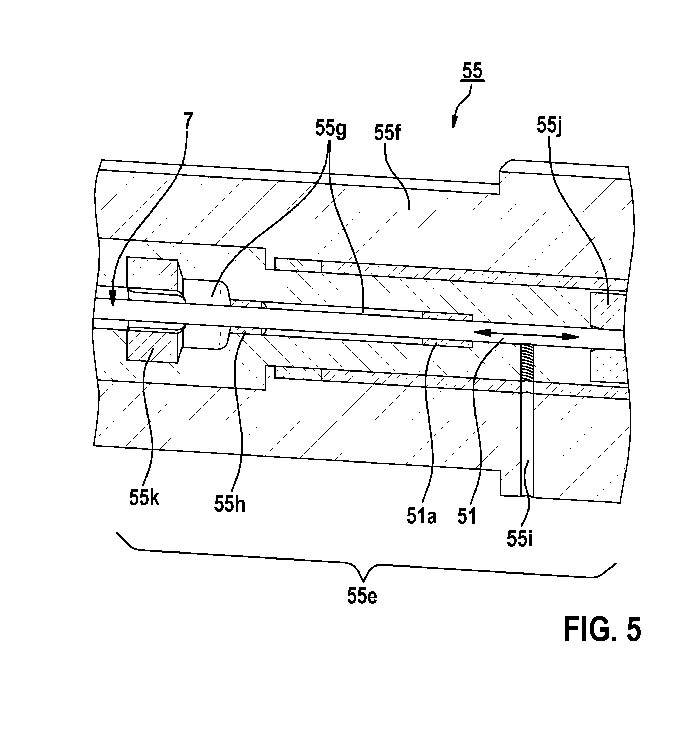

Fig. 5 zeigt den Innenaufbau des Steckeradapters 55, speziell der Dichtungseinheit 55e, in einer perspektivischen Längsschnittdarstellung noch detaillierter. Zentral in der Dichtungseinheit 55e angeordnet ist eine zweiteilige Einfüllkammer 55g, die einen proximalen Kammerabschnitt relativ großen Durchmessers, aber geringer Länge, und einen distalen Kammerabschnitt geringeren Durchmessers, aber relativ großer Länge (nicht gesondert bezeichnet) umfasst. Der distale Kammerabschnitt ist so dimensioniert, dass der Katheterschlauch 51 flüssigkeitsdicht in diesen passt und in ihm längs verschiebbar ist. Sowohl am distalen Ende des Ballonschlauchs 51 als auch zwischen den beiden Abschnitten der Einfüllkammer 55g ist jeweils ein Schlauchstopp 51a bzw. 55h vorgesehen.

Im Adaptergehäuse 55f ist distal vom distalen Ende der Einfüllkammer 55g ein Gewindegang 55i für eine (nicht gezeigte) Madenschraube zur Fixierung der longitudinalen Position des Katheterschlauchs 51 bezüglich des Steckeradapters 55 vorgesehen. Die Fixierungsschraube ist zur longitudinalen Fixierung der Position des gesamten Tools auf dem Katheterkörper (Ballonschlauch) gedacht. Dadurch kann die Position (Abstand) des Fixierballons ausgehend vom Tip (distalem Ende) der Elektrode variiert werden.

Weiter distal von diesem Gewindegang 55i ist im Adapterkörper ein distales Dichtelement 55j angebracht, und auch am proximalen Eingang der Einfüllkammer 55g ist ein zentrales Dichtelement vorgesehen, welches mit Ziffer 55k bezeichnet ist. Hierbei handelt es sich um ein von der Kanüle 7 durchstoßbares und nach Herausziehen der Kanüle selbstverschließendes Dichtelement, welches so ausgestaltet ist, dass es den Flüssigkeitsdruck im System Einfüllkammer-Katheter-Ballon der Fixierungsvorrichtung 5 permanent halten und somit die Außenabmessungen des Ballons und hiermit wiederum dessen Fixierungswirkung dauerhaft gewährleisten kann.

Die Ausführung der Erfindung ist nicht auf die oben beschriebenen Beispiele und hervorgehobenen Aspekte beschränkt, sondern kann ebenso in einer Vielzahl von Abwandlungen erfolgen, die im Rahmen fachmännischen Handelns liegen.

## Patentansprüche

1. Implantierbare medizinische Elektrodenvorrichtung (1), insbesondere kardiovaskuläre Herzschrittmacher-Elektrodenvorrichtung, angepasst zum Einsetzen in ein Blutgefäß, umfassend
eine langgestreckte Elektrodenleitung (3) mit einem schlauchartigen Elektrodenkörper (31), der einen vom proximalen Ende zum distalen Ende durchgehenden Hohlraum (33) mit einem Innendurchmesser aufweist und an dessen distalem Ende eine oder mehrere Elektrode(n) (35a, 35b) und an dessen proximalem Ende ein Elektrodenanschluss (37) vorgesehen sind, und
eine Fixierungsvorrichtung (5) mit einer Ballonkathetereinrichtung, die einen langgestreckten schlauchartigen, hohlen Katheterkörper (51) mit einem Außendurchmesser, der kleiner als der Innendurchmesser des Hohlraums des Elektrodenkörpers ist, und einem inflatierbaren Ballon (53) an oder nahe dem distalen Ende, mit einem im deflatierten Zustand gegenüber dem Innendurchmesser des Hohlraums des Elektrodenkörpers kleineren Außendurchmesser, und einem Fluidanschluss (55a) und Elektrodenanschluss am proximalen Ende, der an den Elektrodenanschluss des Elektrodenkörpers angepasst ist, mit
Verbindungsmitteln (55c, 55d) zur lösbaren Verbindung der Elektrodenleitung mit der Fixierungsvorrichtung und
Dichtungsmitteln (55e) zum fluiddichten Verschluss des Katheterkörpers der Fixierungsvorrichtung in ihrem Gebrauchszustand mit inflatiertem Ballon.

2. Elektrodenvorrichtung nach Anspruch 1, wobei am proximalen Ende der Fixierungsvorrichtung (5) ein Steckeradapter (55) angebracht ist, der innenseitig zur Aufnahme des Elektrodenanschlusses (37) der Elektrodenleitung (3) und außenseitig zum Einführen in eine Elektrodenbuchse eines elektromedizinischen Gerätes, insbesondere Herzschrittmachers, sowie zum Einführen einer Injektionskanüle (7) in den Fluidanschluss (55a) des Katheterkörpers (51) angepasst ist.

3. Elektrodenvorrichtung nach Anspruch 1 oder 2, wobei die Dichtungsmittel (55e) ein formelastisches zylindrisches Dichtelement (55j) umfassen, welches konzentrisch zum Katheterkörper (51) der Ballonkathetereinrichtung (5) angeordnet und von deren proximalem Ende her mit einer Injektionskanüle (7) derart durchstoßbar ist, dass es sich nach dem Entfernen der Injektionskanüle wieder selbsttätig fluiddicht verschließt.

4. Elektrodenvorrichtung nach einem der vorangehenden Ansprüche, wobei Verstellmittel, insbesondere eine in den Elektrodenkörper eingebettete und von dessen Außenumfang zugängliche, radial zum Eindringen in den Hohlraum der Elektrodenleitung und zum Eingriff mit dem schlauchartigen Katheterkörper der hierin eingesetzten Fixierungsvorrichtung ausgerichtete Fixierungsschraube (55i) zur Einstellung der longitudinalen Position der Fixierungsvorrichtung (5) bezüglich der Elektrodenleitung (3) vorgesehen sind.

5. Elektrodenvorrichtung nach einem der Ansprüche 2 bis 4, wobei der äußere Elektrodenanschluss (55a) des Steckeradapters (55) als IS-1 oder IS4 Elektrodenanschluss ausgebildet ist.

6. Elektrodenvorrichtung nach einem der vorangehenden Ansprüche, wobei der Ballon (53) der Ballonkathetereinrichtung (5) aus einem Material aus der Silikon, PUR, ETFG, PTFE und PA umfassenden Gruppe gebildet ist.

7. Fixierungsvorrichtung (5) zur Bildung einer Elektrodenvorrichtung (1) nach einem der vorangehenden Ansprüche.

8. Elektrodenleitung (3), ausgebildet zur Bildung einer Elektrodenvorrichtung (1) nach einem der vorangehenden Ansprüche.

9. Medizinisches Elektrodenimplantationssystem (1, 7), mit einer Elektrodenvorrichtung (1) nach einem der Ansprüche 1 bis 6 sowie
einer Fluidinjektionseinrichtung mit einer Injektionsnadel (7), die einen an den proximalen Fluidanschluss der Ballonkathetereinrichtung angepassten Außendurchmesser hat.
